# EUROPEAN PATENT SPECIFICATION

(11) **EP 2 160 201 B1**
(45) Date of publication and mention of the grant of the patent: **09.04.2014**
(21) Application number: 08767820.7
(22) Date of filing: 20.05.2008
(51) Int. Cl.: A61K 48/00, A61N 1/30

(54) **APPARATUS FOR THE DELIVERY OF POLYNUCLEOTIDE VACCINES TO MAMMALIAN SKIN**
VORRICHTUNG ZUR VERABREICHUNG VON POLYNUKLEOTIDIMPFSTOFFEN AN SÄUGERHAUT
APPAREIL POUR LA DÉLIVRANCE DE VACCINS POLYNUCLÉOTIDIQUES DANS LA PEAU DE MAMMIFÈRES

(30) Priority: 21.05.2007 US 924568 P
(43) Date of publication of application: 10.03.2010
(73) Proprietor: Cellectis, 75013 Paris (FR)
(72) Inventor: WALTERS, Richard, E., Columbia, MD 21045 (US); WALTERS, Derin, C., 125-0041 (JP); KING, Alan, D., Highland, MD 20777 (US); ROOS, Anna-Karin, S-16562 Hasselby (SE)
(74) Representative: Hanson, William Bennett
(86) International application number: PCT/US2008/006442
(87) International publication number: WO 2008/144058

(56) References cited:
- WO-A1-03/089046
- US-A1- 2002 061 589
- US-B1- 6 520 950
- "Easy Vax(TM) Clinical Epidermal Electroporation System - Data Sheet", , 5 February 2007 (2007-02-05), XP055048043, Retrieved from the Internet: URL:http://web.archive.org/web/20070205145 907/http://www.cytopulse.com/pdf/Datasheet Easy Vax.pdf [retrieved on 2012-12-17]
- ROOS ET AL: "Enhancement of cellular immune response to a prostate cancer DNA vaccine by intradermal electroporation", MOLECULAR THERAPY, ACADEMIC PRESS, SAN DIEGO, CA, US, vol. 13, no. 2, 1 February 2006 (2006-02-01), pages 320-327, XP005252891, ISSN: 1525-0016, DOI: 10.1016/J.YMTHE.2005.08.005

## Description

### Technical Field

The present invention relates generally to apparatus for the delivery of polynucleotide vaccines into mammalian skin cells. More specifically, the present invention provides apparatus for the delivery of polynucleotide vaccines into mammalian skin cells using electrical waveforms and electroporation.

### Background Art

For purposes of the present disclosure, the term "pulse interval" means the time from the beginning of one pulse to the beginning of the next pulse.

The following publications are discussed hereinbelow:
U. S. Patent No. 6,010,613;
U. S. Patent No. 6,603,998;
U. S. Patent No. 6,713,291;

"Enhancement of Cellular Immune Response to a Prostate Cancer DNA Vaccine by Intradermal Electroporation", by Roos et al, Molecular Therapy, Vol. 13, No. 2, February 2006, pages 320-327 (referred to herein as Roos et al);

"The effect of pulse repetition frequency on the uptake into electropermeabilized cells in vitro with possible applications in electrochemotherapy", by Pucihar et al, Bioelectrochemistry 57 (2002) pages 167-172 (referred to herein as Pucihar et al).

Vernhes MC, Cabanes PA, Tessie J. Chinease hamster ovary cells sensitivity to localized electrical stress. Bioelectrochemistry and Bioenergetics. 1999, 48:17-25;

Daskalov I, Mudrov N, Peycheva E. Exploring new instrumentation. Parameters for electrochemotherapy. Attacking tumors with bursts of biphasic pulses instead of single pulses. 1999, IEEE Eng. Med. Biol 62-66;

Chang DC, Cell poration and cell fusion using an oscillating electric field. 1989 Biophys J. 56:641-652; and

Tekle E, Astumian RD, Chock PB. Electroporation by using bipolar oscillating electric field: An improved method for DNA transfection of NIH 3T3 cells. 1991 Proc. Natl. Acad. Sci. 88:4230-4234.

U. S. Patent No. 6,010,613 discloses using electroporation with wide interval electrical waveforms, such as provided by PA-4000 System (referred to herein as PulseAgile) of Cyto Pulse, Inc., 810 Cromwell Park Drive, Suite T, Glen Burnie, MD 21061. More specifically, U. S. Patent No. 6,010,613 discloses applying a sequence of at least three single, operator-controlled, independently programmed, DC electrical pulses, to a material, wherein the sequence of at least three DC electrical pulses has one, two, or three of the following characteristics: (1) at least two of the at least three pulses differ from each other in pulse amplitude; (2) at least two of the at least three pulses differ from each other in pulse width; and (3) a first pulse interval for a first set of two of the at least three pulses is different from a second pulse interval for a second set of two of the at least three pulses.

For purposes of the discussions and disclosures herein, the above-mentioned applying a sequence of at least three single, operator-controlled, independently programmed, DC electrical pulses, to a material, with the characteristics (1), (2), and (3) set forth is referred to herein as "PulseAgile".

The specification disclosed in U. S. Patent No. 6,010,613 and the documentation connected with the PulseAgile system provide that the pulse interval is equal to or greater than 0.1 seconds, which is 100 milliseconds. Hereinafter, the PulseAgile generated electrical waveforms which have pulse intervals which are equal to or greater than 100 milliseconds are referred to as "wide interval PulseAgile electrical waveforms" or "slow PulseAgile electrical waveforms".

In U. S. Patent No. 6,010,613, there is no specific evidence presented that administered vaccines have either successful genetic expression of the vaccine or provide improved T-cell response involving improved secretion of good protein resulting from successful genetic expression of the vaccine.

Both U. S. Patent No. 6,603,998 and U. S. Patent No. 6,713,291 disclose the delivery of polynucleotide vaccines to biological cells using the wide interval PulseAgile electrical waveforms or slow PulseAgile electrical waveforms.

Roos et al disclose the use of the wide interval PulseAgile electrical waveforms or slow PulseAgile electrical waveforms to deliver a polynucleotide vaccine into mammalian skin cells. It is also disclosed by Roos et al that successful genetic expression of the polynucleotide vaccine is demonstrated by detection of a genetic marker which expresses luciferase protein. In addition, Roos et al disclose that with the use of the wide interval PulseAgile electrical waveforms or the slow PulseAgile electrical waveforms to deliver a polynucleotide vaccine into mammalian skin cells, there is improved T-cell response involving improved secretion of good protein resulting from successful genetic expression of the polynucleotide vaccine. In Roos et al, T-cell response is represented by PSA-specific IFN(gamma)-producing CD8⁺ T cells.

Aside from the beneficial results disclosed in the Roos et al publication, there are two undesirable results observed by using the slow PulseAgile electrical waveforms. The first undesirable result is that each slow PulseAgile electrical waveform administration protocol took approximately 3.5 seconds. Since administration employing the use of needles penetrating into mammalian skin causes discomfort or pain, for such 3.5 second administration protocol, the mammal would have to endure the discomfort or pain for approximately 3.5 seconds.

The second undesirable result disclosed in Roos et al is that each slow PulseAgile electrical waveform causes a perceptible muscle contraction. The muscle contraction itself can also cause discomfort or pain. Normally, for an administration of a polynucleotide vaccine, plural pulsed waveforms would be applied to a mammal. Therefore, plural muscle contractions, with plural additional muscle discomfort or pain, would take place with such slow PulseAgile electrical waveforms.

A document from Cyto Pulse, Inc. entitled Data. Sheet/Easy Vax^{™} Clinical Epidermal Electroporation System, Jan 2007, describes a portable transdermal micro-needle array system in which pulse intervals can range from 0.200 to 1000 ms.

Pucihar et al disclose that, before their publication date in 2002, electrical pulses have been used in combination with chemotherapeutic agents to treat cancerous cells. The earlier electrical pulses have had a frequency of 1 Hz, whereby each pulse produced a related tetanic contraction (muscle contraction). It is noted that 1 Hz translates to 1000 milliseconds per cycle. The discussed electrical pulse protocols are all pulse sequences that have pulses of uniform pulse amplitude, uniform pulse width, and uniform pulse interval. The chemotherapeutic agents include small nonpermeant hydrophilic molecules. The disclosures of the research conducted by Pucihar et al relate to in vitro (not in vivo) experiments with cancerous cell being treated with Lucifer Yellow, which is a small nonpermeant hydrophilic molecule. The disclosures of the research conducted by Pucihar et al explore various pulse repetition frequencies in order to exceed the frequency of tetanic contraction (so that successive muscle contractions fuse into smooth motion). There is a statement in Pucihar et al that with a frequency of excitation of 40 Hz or faster, successive muscle contractions fuse into smooth motion. The 40 Hz pulse frequency employs pulses of uniform pulse amplitude, uniform pulse width, and uniform pulse interval. It is noted that 40 Hz translates to 25 milliseconds per cycle.

Vernhes et al disclose that viability and permeability of CHO cells electroporated in vitro were high over an electroporation pulse frequency range of 0.5 to 100 HZ.

Daskalov et al disclose that eight bipolar pulses delivered to tumor cells in vivo produced a similar response to electrochemotherapy when delivered at 1 HZ and 1kHZ.

Chang discloses that high frequency sinusoidal waveforms delivered as short pulses efficiently electroporated COS-M-6 cells in vitro.

Tekle et al disclose that unipolar or bipolar rectangular wave pulses delivered at frequencies ranging from 60 kHZ to 1 MHZ efficiently transfected NIH 3T3 cells in vitro.

There is no disclosure in any of Pucihar, Vernhes et al, Daskalov et al, Chang, or Tekle et al which states any relationship to polynucleotide vaccination, to successful genetic expression of a polynucleotide vaccine, or to improved T-cell response involving improved secretion of a desired protein resulting from successful genetic expression of the polynucleotide vaccine.

In view of the above, it would be desirable to provide a method and apparatus for the delivery of polynucleotide vaccine into mammalian skin cells which takes less than 3.5 seconds to administer the polynucleotide vaccine.

In addition, it would be desirable to provide a method and apparatus for the delivery of polynucleotide vaccines into mammalian skin cells which applies plural PulseAgile electrical waveforms to the mammalian skin and only causes one muscle contraction for the plural applied electrical waveforms.

Administration of a polynucleotide vaccine, to be successful, must give evidence of successful genetic expression of the administered polynucleotide vaccine. Moreover, to be successful, the genetic expression of the administered polynucleotide must give evidence of providing a desired protein which results from the successful genetic expression of the polynucleotide vaccine.

Thus, while the foregoing body of prior art indicates it to be well known to use electroporation apparatuses, the prior art described above does not teach or suggest a method and apparatus for the delivery of polynucleotide vaccines into mammalian skin cells which has the following combination of desirable features: (1) provides a method and apparatus for the delivery of polynucleotide vaccine into mammalian skin cells which takes less than 3.5 seconds to administer the polynucleotide vaccine; (2) applies plural PulseAgile electrical waveforms to the mammalian skin and only causes one muscle contraction for the plural applied electrical waveforms; (3) gives evidence of successful genetic expression of the administered polynucleotide vaccine; and (4) gives evidence of providing a desired protein which results from the successful genetic expression of the polynucleotide vaccine.

The foregoing desired characteristics are provided by the unique apparatus for the delivery of polynucleotide vaccines into mammalian skin cells of the present invention as will be made apparent from the following description thereof. Other advantages of the present invention rover the prior art also will be rendered evident.

### Disclosure of Invention

In accordance with the invention, an apparatus is provided for the delivery of polynucleotide vaccines into mammalian skin cells, according to claim 1.

With one embodiment of the apparatus of the invention, the polynucleotide vaccine is applied to the skin prior to contacting the skin with the electrode. This can be accomplished by using a hypodermic needle.

With another embodiment of the apparatus of the invention, the polynucleotide vaccine is pre-coated on the electrode and is applied to the skin at the same time the electrode is contacted with the skin.

The apparatus that provides fast PulseAgile electrical waveforms or narrow interval PulseAgile electrical waveforms and that employs any suitable electrode for application to mammalian skin is made by Cyto Pulse, Inc., 810 Cromwell Park Drive, Suite T, Glen Burnie, MD 21061, and is known by the name "Derma Vax".

More information about the Cyto Pulse, Inc. "Derma Vax" system is in the following publication: a Data Sheet entitled "Derma vax ™ Clinical Evaluation Intra-dermal System", which available to the public on the Internet at the following URL address -- www.cytopulse.com/dna_vaccine.shtml, followed by a click on the link entitled "Derma Vax Data Sheet (99 Kb)". The Data Sheet itself is located at the following URL address -- http://www.cytopulse.com/pdf/Datasheet%20Derma%20Vax.pdf.

The apparatus that provides fast PulseAgile electrical waveforms or narrow interval PulseAgile electrical waveforms and that employs a pre-coated electrode suitable for application to mammalian skin is also made by Cyto Pulse, Inc. and is known by the name "Easy Vax".

The apparatus that provides fast PulseAgile electrical waveforms or narrow interval PulseAgile electrical waveforms is also made by Cyto Pulse, Inc. and is known by the name "CCEP-40 Waveform Generator". As stated above, specifications for the "CCEP-40 Waveform Generator" are provided in the Data Sheet entitled "Derma Vax ™ Clinical Evaluation Intra-dermal System" mentioned above.

The above brief description sets forth rather broadly the more important features of the present invention in order that the detailed description thereof that follows may be better understood, and in order that the present contributions to the art may be better appreciated. There are, of course, additional features of the invention that will be described hereinafter and which will be for the subject matter of the claims appended hereto.

It is therefore an object of the present invention to provide a new and improved apparatus for the delivery of polynucleotide vaccines into mammalian skin cells which takes less than 3.5 seconds to administer the polynucleotide vaccine.

Still another object of the present invention is to provide a new and improved apparatus for the delivery of polynucleotide vaccines into mammalian skin cells that applies plural PulseAgile electrical waveforms to the mammalian skin and only causes one muscle contraction for the plural applied electrical waveforms.

Yet another object of the present invention is to provide a new and improved apparatus for the delivery of polynucleotide vaccines into mammalian skin cells which gives evidence of successful genetic expression of the administered polynucleotide vaccine.

Even another object of the present invention is to provide a new and improved apparatus for the delivery of polynucleotide vaccines into mammalian skin cells that gives evidence of providing a desired protein which results from the successful genetic expression of the polynucleotide vaccine.

These together with still other objects of the invention, along with the various features of novelty which characterize the invention, are pointed out with particularity in the claims annexed to and forming a part of this disclosure. For a better understanding of the invention, its operating advantages and the specific objects attained by its uses, reference should be had to the accompanying drawings and descriptive matter in which there are illustrated preferred embodiments of the invention.

### Brief Description of Drawings

The invention will be better understood and the above objects as well as objects other than those set forth above will become more apparent after a study of the following detailed description thereof. Such description makes reference to the annexed drawing wherein:
Fig. 1 is a graph illustrating a comparison of luciferase expression resulting from the application of fast PulseAgile electrical waveforms versus the application of slow PulseAgile electrical waveforms for delivery of luciferase plasmid with electroporation using "Derma Vax" equipment.
Fig. 2 a graph illustrating a comparison of T cell response to vaccination using Dengue 1 DNA vaccine, resulting from the application of fast PulseAgile electrical waveforms versus the application of slow PulseAgile electrical waveforms for delivery of the vaccine with electroporation using "Derma Vax" equipment.
Fig. 3 is a graph illustrating a comparison of luciferase expression resulting from the application of fast PulseAgile electrical waveforms versus the application of slow PulseAgile electrical waveforms for delivery of luciferase plasmid with electroporation using "Derma Vax" equipment.

### Modes for Carrying Out the Invention

A method and apparatus are disclosed for the delivery of polynucleotide vaccines into mammalian skin cells, and with reference to the drawings, said method and apparatus are described below.

Specifications for "Derma Vax" and "CCEP-40 Waveform Generator" are as follows:

### DERMA VAX Specifications

### Operation

### Mode 1 - Vaccine Delivery by trained health professional

Touch Screen
   Opening Screen for parameter entry
      Patient ID entry
      Vaccine ID entry
      Electrode ID entry
   Vaccination Screen
      SKIN - measure skin resistance every second and display
      READY - turn on high voltage power supply
      START - start pulsing
      DONE - vaccination completed

### Mode 2 - Setup by trained IT specialist

Pulse parameters
Download data files

### Delivery Electrode

Vaccine delivery volume --
   2 blebs x 25 µl each IDA-4-6
   2 blebs x 50 µl each IDA-6-6
Delivery target -- skin/dermis Electrode
   Handle -- Reusable with alcohol cleaning
   Tip --
      Sterile
      Single packaged
      Disposable

| | IDA-4-4 | IDA-4-6 | IDA-6-6 |
|---|---|---|---|
| Row spacing | 4 mm | 4 mm | 6 mm |
| Needles/row | 4 | 6 | 6 |
| Needle spacing | 1.5 mm | 1.5 mm | 1.5 mm |
| Needle diameter | 0.3 mm | 0.3 mm | 0.3 mm |
| Needle length | 2 mm | 3 mm | 3 mm |
| V/d maximum | 2500 v/cm | 2500 v/cm | 1667 v/cm |

### CCBP-40 Waveform Generator

### Pulsing

Skin resistance pulsing -- 4 µs at 5 volts every second Pulse Protocol Parameters

Parameters in a Group

| | | |
|---|---|---|
| Pulse Width | 50 µs to 1 ms | 50 to 1000 volts |
| | 50 µs to 10 ms | 50 to 300 volts |
| Pulse current trip | | 26 amps |
| Load Range | | 15 to 1500 ohms |
| Number of pulses | | 1 to 10 |
| Maximum Duty Cycle | | 50% |
| Interval | | 200 µs to 1 sec (pulse start to pulse start) |
| Number of Groups | | 3 |

### Pulse Measurement

Internal Digitizer

| | |
|---|---|
| Levels | 12 bit |
| Samples | Pulse width/8 minimum 100 µs |

### Data stored internally and on external USB Key

Data Types
Raw data: DV<Date>.xml
Log Data DV<Date>.txt
CSV Data DV<Date>.csv

All data automatically stored in internal memory and may be downloaded to an external USB Key

Maximum Data Logs stored and retrievable from internal flash memory > 20,000

### Front Panel

Computer

| | |
|---|---|
| Operating System | Windows™ Mobile 6.0 |
| Interface | Touch screen |

Line/Mains Switch with illumination
Emergency Stop Button (resets computer to ready state)
Touch Screen

| | |
|---|---|
| USB Ports | 2 |
| Electrode | connector Fischer Series 4032 |

### Back Panel

| | |
|---|---|
| Power Entry | IEC 320 |
| Ethernet | RJ45 |

### Electrical and Mechanical

| | |
|---|---|
| CCEP-40A Cabinet with handle | |
| 32 mm w x 20 mm h x 40 mm 1 | |
| 12.6 in w x 7.9 in h x 15.7 in 1 | |
| Weight | 25 pounds, 11.3 kg |
| Operating temperature | 10 to 40 °C |
| Mains Voltage | 100 to 250 vac |
| Fuse | 5 A slo blo, 5 mm x 20 mm |
| Power reserve | > 5 minutes after power fail |

Experiments for carrying out the disclosed method employing apparatus of the invention for the delivery of polynucleotide vaccines into mammalian skin cells are set forth below.

### Experiment 1

### PURPOSE AND SCOPE

The purpose of this experiment is to compare fast PulseAgile electrical waveforms (using the Cyto Pulse "Derma Vax" system) versus slow PulseAgile electrical waveforms (using the Cyto Pulse PA-4000 system). The new Derma Vax system can deliver pulses more rapidly than the PA-4000.

### BACKGROUND

Dr. Anna-Karin Roos published at least two waveforms that induced good luciferase expression in the skin of mice. The system used was the PA-4000, and slow PulseAgile electrical waveforms were employed. New capabilities have been engineered into the Derma Vax system which employs the "CCEP-40 Waveform Generator". One significant difference is that the Derma Vax system can deliver pulses with shorter pulse intervals. That is, with the "Derma Vax" system, pulse intervals of less than 100 milliseconds can be provided. This experiment will evaluate the effect on in vivo luciferase expression using fast PulseAgile electrical waveforms.

### APPROACH

Plasmid used: gWizLuciferase from Aldeveron at 5 mg/ml diluted to 0.5 mg/ml in sterile PBS.

System: Derma Vax #F2LQ2608851

Electrode: Intradermal Array (4 mm gap, 6 needles per row, 2 rows) parallel row electrode.

Injections: Mice were restrained using a 50 ml conical tube modified with breathing holes. The mouse was inserted head first into the tube. The tail was draped over my left index finger. A small patch of hair was removed on the base of the tail using small scissors. Using a 27 gauge, 0.5 in needle on a tuberculin syringe, a 20 microliter intradermal injection was made on the right side of the base of the tail and sacrum. The site was marked using a Sharpee pen. The rows of needles were inserted around the injection site with the electrode gap oriented left to right and therefore the rows were aligned cranially and caudally. The selected electroporation protocol was initiated and the needles removed. This process was repeated on the left side of the sacrum.

Groups (shown as cages in results). All times are shown in milliseconds

| Protocols | Cage 1 | Cage 2 |
|---|---|---|
| V/d 1 | 1125 | 1125 |
| V1 | 450 | 450 |
| PW 1 | 0.05 | 0.05 |
| #1 | 1 | 1 |
| PI1 | 300 | 0.2 |
| V/d2 | 1125 | 1125 |
| V2 | 450 | 450 |
| PW 2 | 0.05 | 0.05 |
| # 2 | 1 | 1 |
| PI2 | 500 | 100 |
| V/d 3 | 275 | 275 |
| V3 | 110 | 110 |
| PW 3 | 10 | 10 |
| #3 | 8 | 8 |
| PI 3 | 300 | 20 |

Mice were returned to their cages.

After 18-24 hours, the mice were euthanized using CO2 inhalation. Tissue from each of the two sites was incised using a 6 mm punch biopsy. Subcutaneous tissue was removed using scissors and the skin with subcutaneous tissue was added to 1 mi of lysis buffer. The sample was kept on ice until the assay.

Tissues were homogenized using a model IKA tissue homogenizer. A 50 microliter sample of the 1 ml homogenate was added to a white assay plate. Standards were made by diluting a know amount of luciferase with lysis buffer using a three fold dilution series. 50 microliter reagent A of the luciferase assay kit was added to each well. The plate was added to the 96 well luminometer. 50 microliter of reagent B was added and the resulting light was measured over one second.

Data was exported to an Excell spreadsheet for data analysis.

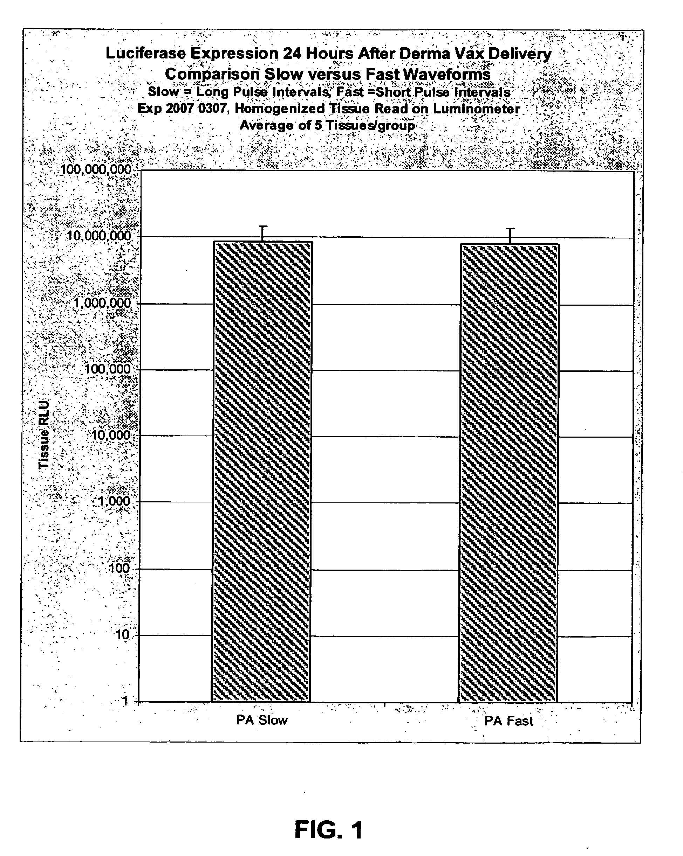

Reference is made to Fig. 1 in the drawings for a graphical representation of the results. There is a statistical equivalence of genetic expression between fast and slow electrical waveform protocols.

### RESULTS

| | | ng/site |
|---|---|---|
| | Cage 1 | Cage 2 |
| | PA Slow | PA Fast |
| | 22 | 43 |
| | 464 | 510 |
| | 486 | 283 |
| | 180 | 267 |
| | 197 | 168 |
| Mean | 270 | 254 |
| SD | 200 | 172 |
| CV | 74 | 68 |

It is a surprising and unexpected result that electroporation of a polynucleotide vaccine into mammalian skin cells along with successful gene expression can occur with fast PulseAgile electrical waveforms having a pulse interval of less than 100 milliseconds.

It is an even greater surprising and unexpected result that electroporation of a polynucleotide vaccine into mammalian skin cells, along with successful gene expression, can occur with fast PulseAgile electrical waveforms having a pulse interval of a few milliseconds. Conventionally, it would be expected that the time constant of pulse intervals of only a few milliseconds would be too low for successful electroporation.

### Experiment 2

### PURPOSE AND SCOPE

The purpose of this experiment is to compare T cell responses induced by DNA immunization using fast PulseAgile electrical waveforms (using the Cyto Pulse "Derma Vax" system) versus slow PulseAgile electrical waveforms (using the Cyto Pulse PA-4000 system). The new Derma Vax system can deliver pulses more rapidly than the PA-4000. More specifically, the purpose of this study is to compare T cell responses induced by DNA immunization using Pulse Agile Derma Vax delivery with Dengue 1 plasmids expressing prM-E and NS1-NS3.

### BACKGROUND

Dr. Anna-Karin Roos published at least two waveforms that induced good luciferase expression in the skin of mice. The system used was the PA-4000, and slow PulseAgile electrical waveforms were employed. New capabilities have been engineered into the Derma Vax system which employs the "CCEP-40 Waveform Generator". One significant difference is that the Derma Vax system can deliver pulses with shorter pulse intervals. That is, with the "Derma Vax" system, pulse intervals of less than 100 milliseconds can be provided. This experiment will evaluate the effect on in vivo T cell responses using fast PulseAgile electrical waveforms.

### APPROACH

Plasmid used: Dengue 1 prM-E and Dengue 1 NS1-NS3 at 5 mg/ml each diluted to 0.5 mg/ml in the same sterile PBS.

System: Derma Vax #07-0215DV

Electrode: Intradermal Array (4 mm gap, 6 needles per row, 2 rows) parallel row electrode.

Injections: Mice were restrained using a 50 ml conical tube modified with breathing holes. The mouse was inserted head first into the tube. The tail was draped over my left index finger. A small patch of hair was removed on the base of the tail using small scissors. Using a 27 gauge, 0.5 in needle on a tuberculin syringe, a 20 µl intradermal injection was made on the right side of the base of the tail and sacrum. The rows of needles were inserted around the injection site with the electrode gap oriented left to right and therefore the rows were aligned cranially and caudally. The selected electroporation protocol was initiated and the needles removed. This process was repeated on the left side of the sacrum

### Groups (shown as cages in results). All times are shown in milliseconds

| Protocols | Group P | Group Q | Control |
|---|---|---|---|
| V/d 1 | 1125 | 1125 | 0 |
| V1 | 450 | 450 | 0 |
| PW 1 | 0.05 | 0.05 | 0 |
| # 1 | 1 | 1 | 0 |
| PI 1 | 0.2 | 300 | 0 |
| V/d 2 | 1125 | 1125 | |
| V2 | 450 | 450 | |
| PW 2 | 0.05 | 0.05 | |
| # 2 | 1 | 1 | |
| PI 2 | 30 | 300 | |
| V/d 3 | 275 | 275 | |
| 3 | 110 | 110 | |
| PW 3 | 10 | 10 | |
| # 3 | 8 | 8 | |
| PI 3 | 20 | 100 | |

Mice were returned to their cages.

At 2 weeks after immunization, mice were euthanized using CO2 inhalation and the spleens were collected for intracellular cytokine assay.

### RESULTS

Results show below are percent of CD8 positive cells that are gamma interferon positive.
Results are shown with background from un-immunized animals subtracted.

| | Fast (Group P) | Slow (Group Q) |
|---|---|---|
| | 6.42 | 4.03 |
| | 5.38 | 6.11 |
| | 4.35 | 3.36 |
| | 4.28 | 2.75 |
| | 2.06 | 2.16 |
| Mean | 4.50 | 3.68 |
| SD | 1.62 | 1.53 |

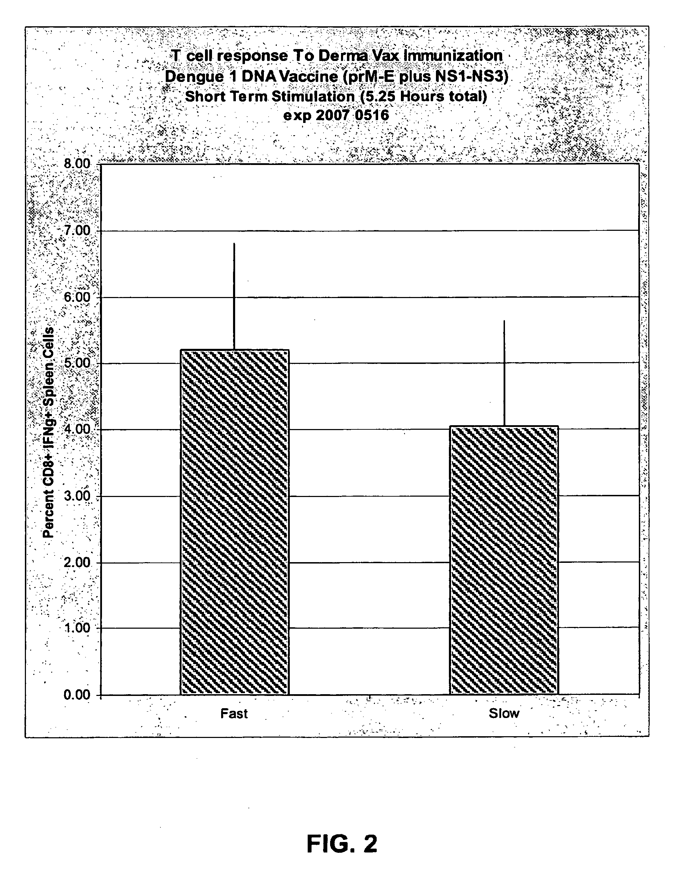

Reference is made to Fig. 2 in the drawings for a graphical representation of the test results. In this respect, by conducting a Student's T test, the test results show a statistically insignificant difference between fast and slow electrical waveform protocols. In this respect, there is a statistical equivalence of T cell enhancement between fast and slow electrical waveform protocols.

### CONCLUSIONS

T cell responses induced by fast PulseAgile electrical waveforms with the "Derma Vax" system are equivalent to those induced by slow PulseAgile electrical waveforms with the "Derma Vax" system with in vivo electroporation.

**TABLE I**

| Perceptible muscle contractions are reduced by electroporation using fast PulseAgile electrical waveforms in contrast with slow PulseAgile electrical waveforms. | | |
|---|---|---|
| Parameter | Fast Pulse Agile | Slow Pulse Agile |
| Pulses Delivered | 10 | 10 |
| Total Delivery Time | 0.23 Seconds | 3.5 Seconds |
| Perceptible Muscle Contractions | 1 | 10 |

Clearly, with fast PulseAgile electrical waveforms (as compared with slow PulseAgile electrical waveforms), delivery time is much less than 3.5 seconds, and only 1 muscle contraction is perceived, even when 10 pulses are delivered.

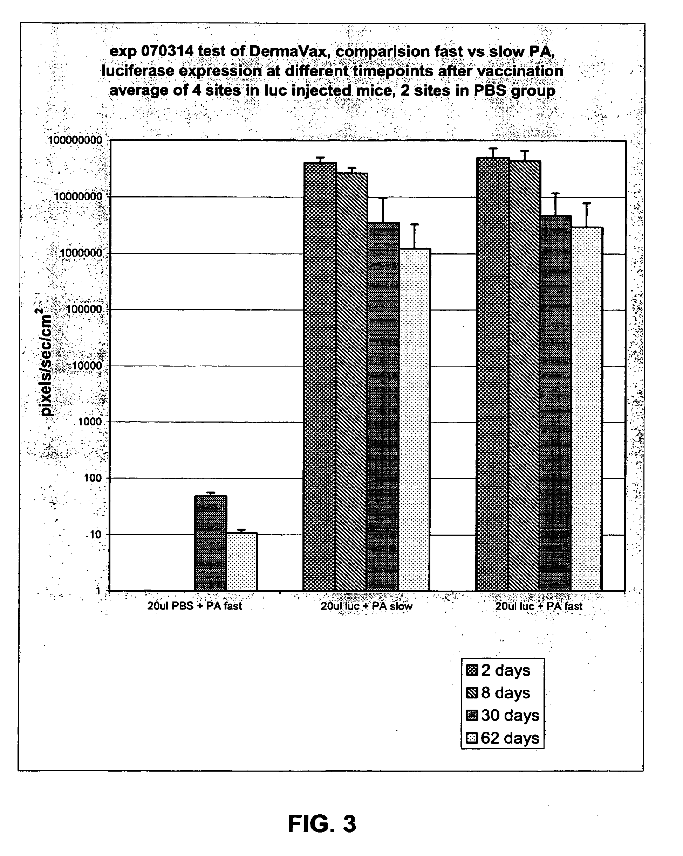

With respect to Fig. 3, DNA delivery (DNA being a polynucleotide) was carried out as follows.

Mice were anesthetized with 4 % isoflurane (Baxter Medical AB, Kista, Sweden) and maintained at 2-2.5 % isoflurane in a mask during immunizations. 20 µg DNA in PBS was injected intradermally on each flank, near the base of the tail, using a 29 G insulin grade syringe (Micro-Fine U-100, BD Consumer Healthcare, Franklin Lakes, NJ).

Subsequently, a needle array electrode was placed over the raised skin area of injection and voltage was applied (2 pulses, 1125 V/cm, 50 µsec + 8 pulses, 275 V/cm, 10 msec). Pulse intervals were varied to make fast and slow PulseAgile protocols.

The needle array electrode used was the Cyto Pulse Intradermal array (four needle, 4 mm gap, two rows) (Cyto Pulse Sciences, Inc. Glen Burnie, MD). Electroporation was performed using the Derma Vax Electroporation System (Cyto Pulse Sciences, Inc.).

It is apparent from the above that the present invention accomplishes all of the objects set forth by providing a new and improved apparatus for the delivery of polynucleotide vaccines into mammalian skin cells that may advantageously be used and which takes less than 3.5 seconds to administer the polynucleotide vaccine. With the invention, a method and apparatus for the delivery of polynucleotide vaccines into mammalian skin cells are provided which applies plural PulseAgile electrical waveforms to the mammalian skin and only causes one muscle contraction for the plural applied electrical waveforms. With the invention, apparatus for the delivery of polynucleotide vaccines into mammalian skin cells are provided which gives evidence of successful genetic expression of the administered polynucleotide vaccine. With the invention, apparatus for the delivery of polynucleotide vaccines into mammalian skin cells are provided which give evidence of providing a desired protein which results from the successful genetic expression of the polynucleotide vaccine.

As to the manner of usage and operation of the instant invention, the same is apparent from the above disclosure, and accordingly, no further discussion relative to the manner of usage and operation need be provided.

## Claims

1. An apparatus for the delivery of polynucleotide vaccines into mammalian skin cells, comprising:
an electrical waveform generator programmed to apply a sequence of at least three single, operator-controlled, independently programmed electrical pulses, wherein the sequence of at least three pulses has one, two, or three of the following characteristics: (1) at least two of the at least three pulses differ from each other in pulse amplitude; (2) at least two of the at least three pulses differ from each other in pulse width; and (3) a first pulse interval for a first set of two of the at least three pulses is different from a second pulse interval for a second set of two of the at least three pulses, and
an electrode connected to said electrical waveform generator, wherein said electrode is suitable for contacting skin;
**characterised in that** said electrical pulses have pulse intervals that are less than 100 milliseconds.

2. The apparatus of claim 1 wherein the apparatus is adapted to deliver, into mammalian skin cells, a polynucleotide vaccine that has been applied to the skin prior to contacting the skin with the electrode.

3. The apparatus of claim 1 wherein the apparatus is adapted to deliver, into mammalian skin cells, a polynucleotide vaccine that is pre-coated on the electrode and that is applied to the skin at the same time the electrode is contacted with the skin.

## Patentansprüche

1. Apparat zum Einbringen von Polynukleotid-Vakzinen in Hautzellen von Säugern umfassend einen elektrischen Funktionsgenerator programmiert zum Applizieren einer Sequenz von mindestens drei einzelnen, Benutzergesteuerten, unabhängig programmierten elektrischen Pulsen, wobei die Sequenz der mindestens drei Pulse ein, zwei, oder drei der folgenden Eigenschaften aufweist: 1. mindestens zwei der mindestens drei Pulse unterscheiden sich voneinander in ihrer Pulsamplitude; 2. mindestens zwei der mindestens drei Pulse unterscheiden sich voneinander in ihrer Pulsweite; und 3. ein erster Pulsintervall für ein ersten Set von zwei von mindestens drei Pulsen unterscheidet sich von einem zweiten Pulsintervall für ein zweites Set von zwei von den mindestens drei Pulsen, und eine Elektrode verbunden mit dem elektrischen Funktionsgenerator, wobei diese Elektrode geeignet ist zum Kontaktaufnehmen mit der Haut, **dadurch gekennzeichnet, dass** die elektrischen Pulse Pulsintervalle aufzeigen, die kleiner als 100 ms sind.

2. Apparat nach Anspruch 1, wobei der Apparat angepasst ist zum Einbringen in Hautzellen von Säugern eines Polynukleotidvakzines, das auf die Haut aufgebracht wurde, bevor die Haut mit der Elektrode in Kontakt gebracht wird.

3. Apparat nach Anspruch 1, wobei der Apparat angepasst ist zum Einbringen in Hautzellen von Säugern eines Polynukleotidvakzines, das auf die Elektrode vorbeschichtet ist und das zur gleichen Zeit auf die Haut aufgebracht wird, wenn die Elektrode mit der Haut in Kontakt gebracht wird.

## Revendications

1. Appareil pour l'administration de vaccins polynucléotidiques dans des cellules cutanées de mammifères, comprenant
un générateur de formes d'ondes électriques programmé pour appliquer une séquence d'au moins trois impulsions électriques simples contrôlées par un opérateur et indépendamment programmées, la séquence d'au moins trois impulsions présentant une, deux ou trois des caractéristiques suivantes : (1) au moins deux des au moins trois impulsions diffèrent l'une de l'autre en amplitude ; (2) au moins deux des au moins trois impulsions diffèrent l'une de l'autre en largeur ; et (3) un premier intervalle d'impulsion pour un premier ensemble de deux des au moins trois impulsions diffère d'un second intervalle d'impulsion pour un second ensemble de deux des au moins trois impulsions, et
une électrode reliée audit générateur d'ondes électriques, ladite électrode étant adaptée pour venir en contact avec la peau ;
**caractérisé en ce que** lesdites impulsions électriques ont des intervalles inférieurs à 100 millisecondes.

2. Appareil selon la revendication 1, dans lequel l'appareil est adapté pour administrer, dans des cellules cutanées de mammifères, un vaccin polynucléotidique qui a été appliqué sur la peau avant de mettre la peau en contact avec l'électrode.

3. Appareil selon la revendication 1, dans lequel l'appareil est adapté pour administrer, dans des cellules cutanées de mammifères, un vaccin polynucléotidique qui est préalablement déposé sur l'électrode et qui est appliqué sur la peau en même temps que l'électrode est en contact avec la peau.
